(19) **Europäisches Patentamt / European Patent Office / Office européen des brevets**

(11) **EP 3 575 608 B1**

(12) **EUROPEAN PATENT SPECIFICATION**

(45) Date of publication and mention of the grant of the patent:
**01.06.2022 Bulletin 2022/22**

(21) Application number: **19460024.3**

(22) Date of filing: **30.04.2019**

(51) International Patent Classification (IPC):
**F04D 13/06** (2006.01)   **F04D 29/048** (2006.01)
**F04D 3/00** (2006.01)   **A61M 60/00** (2021.01)
**F04D 29/041** (2006.01)

(52) Cooperative Patent Classification (CPC):
**F04D 13/0646; A61M 60/135; A61M 60/205; F04D 3/005; F04D 29/041;** F04D 29/0476

(54) **AXIAL FLOW BLOOD PUMP WITH PARTICULAR DIAMETER RATIOS**

AXIALSTRÖMUNG-BLUTPUMPE MIT BESONDEREN DURCHMESSER-VERHÄLTNISSEN

PUMPE À SANG À FLUX AXIAL AVEC DES RAPPORTS DE DIAMÈTRES SPÉCIFIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.05.2018 PL 42575818**

(43) Date of publication of application:
**04.12.2019 Bulletin 2019/49**

(73) Proprietor: **Centrum Techniki Okretowej S.A.**
**80-392 Gdansk (PL)**

(72) Inventors:
• **Borowiecki, Bartosz**
  **80-768 Gdansk (PL)**

• **Dawidowska, Kinga**
  **77-100 Bytów (PL)**
• **Ditrich, Michal**
  **80-298 Gdansk (PL)**
• **Hoffman, Pawel**
  **80-393 Gdansk (PL)**
• **Siondalski, Piotr**
  **83-050 Pregowo (PL)**
• **Leszek, Wilczynski**
  **80-419 Gdansk (PL)**

(56) References cited:
**WO-A1-2017/004175   US-A- 5 211 546**

## Description

The subject of the invention is an axial-flow pump with a hybrid drive-bearing unit for its rotor, in particular supporting heart function. The unit according to the invention can be used as a main element of a heart-assist pump, both as a device implanted within the body of the patient, as well as an extracorporeal device to treat insufficiency of one of heart ventricles. The invention can also be used in other axial pumps, in particular in small axial pumps.

American patent disclosure US6527521 describes a magnetically-driven axial-flow pump provided with magnetic and/or electromagnetic bearings, comprising of a housing body with a fixed duct and a rotor installed in said duct provided inside the housing. The pump is driven by a set of coils generating a rotary magnetic field, which influences with drive magnets of the rotor and thus allows the rotor to rotate around its axis/around the flow duct axis. Contactless bearing system of the rotor is provided using a system of passive magnetic bearings including magnets located inside the rotor and magnets located inside the pump housing and/or active magnetic bearings, including coils placed around the circumference of the pump housing and magnets placed inside the housing. There are many components in the disclosed bearing system of the axial-flow pump rotor, such as: coil or coils generating magnetic field, power terminal providing power supply to the coil or coils, a system controlling power supply of the coil or coils, magnets or a single magnet, which may increase the risk of pump malfunction, potentially disqualifying the solution from use in a responsible design, such as e.g. pump supporting inefficient heart functions.

The documentation also discloses that magnets with non-standard geometry or non-standard magnetisation method are used in the bearing system, or multiple small magnets, which means that execution accuracy of such magnets may not be adequate for use in a rotary pump system supporting heart functions. Such a pump is characterised by small dimensions and rotation speeds in the range between several and several tens of thousands revolutions per minute.

Geometry of the flow duct presented in that disclosure is characterised with variable internal diameter of the housing and the internal diameter of the duct along its longitudinal axis, which is difficult to provide as a divided model without a minimum discontinuity of the surface contacting the working medium. This may deteriorate thrombogenic properties of the pump if used in a pump design supporting heart functions. The proposed geometry of the flow duct is caused by the necessity of using an active and/or a passive magnetic bearing protecting the rotor against mechanical contact with the flow system and protecting the rotor against sliding out of the working area in the post-axial direction.

Patent application WO2007003351 discloses an axial pump with spiral-shaped rotor blades. The pump is provided with a longitudinal rotor placed in a housing with an inlet and an outlet. The rotor is provided with an outlet including a spiral transferring elements, the pitch of which increases towards the outlet. The electromagnetic drive is used to propel the rotor and the stator made of ferromagnetic material provided as a metal sheet pack, as a monolithic element or as printed material, is placed inside a housing, wherein the stator cooperates with the magnetically-operated rotor. In order to protect the rotor against post-axial sliding, additional locking magnets are provided inside the housing and variable geometry of the duct, in which the pump rotor is installed, is maintained.

The publication "Opracowanie konstrukcji osiowej pompy wspomagania serca" [Wilczyński L., Ditrich M., Hoffmann P., Dawidowska K., Borowiecki B., Górski W., Jakubowski P., Siondalski P., Smolenski R.; www.researchgate.net; full text April 02, 2015] discloses an implantable axial-flow pump intended to support the left cardiac ventricle. This pump was designed at Centrum Techniki Okrętowej S.A. in Gdansk. Tests included numerical calculations of flow through the pump, calculations related to the design of the contactless drive system of the pump and of the magnetic suspension of its rotor, as well as a range of experiments related to pump operation in *in vitro* conditions. The ring-shaped stator provided with teeth with coils wound on them or the stator without teeth, provided as a ring with coils located on the inside, is placed on a cylindrical bushing of the flow duct, inside which a snug fitted rotor provided with blades, installed on the inside on the cylindrical rotor body, is provided. Permanent, neodymium-based drive magnets are installed on the body circumference. Duct-type flow caverns may be provided on the surface of the rotor body.

Designs of many hydrodynamic bearings used in heart-supporting pumps complicate the technological process due to the additional complication of a pump geometry and may increase blood lysis, a phenomenon undesirable in heart support systems, because of the shape of surfaces generating bearing forces. An example of such a solution is disclosed in the patent document US8790236, where protection of the rotor of an axial blood pump against the undesirable sliding in the post-axial direction has also been achieved thanks to a variable cross-section of the duct inside the housing.

Further prior art documents representing the background of the present invention are US 5 211 546 A and WO 2017/004175 A1.

The objective of the invention is to develop a solution without these disadvantages.

The invention is a pump as defined by claim 1.

As specified in claim 2, the rotor body is preferably provided with circumferentially bevelled edges.

The required drive torque and stable bearing forces acting on the rotor in the post-axial direction are generated in the unit according to the invention, protecting the rotor against leaving the working range, as well as in the transverse direction, using the fewest possible design elements. An advantage of the unit according to the invention includes the use of a rotor drive system in the bearing system in the transverse direction and in the bearing system, in the post-axial direction. The contactless bearing system operating in the transverse direction is formed by a liquid film formed in the gap between the internal surface of the flow duct and the external surface of the rotor. Generatrices of these surfaces are matching their shapes or exist as parallel sections. The bearing system is formed in the post-axial direction as a result of magnetic interaction forces between the stator and the drive magnets or a single drive magnet provided in the form of a ring. Connections between blade tips and the internal surface of the rotor body allowed their active surface area to be increased, resulting in the desired pressure difference in the rotor at relatively low rotation speed, as well as allowed the distance between stator coils and the rotor body to be decreased.

Design parameters of the engine are also directly related to design parameters of the post-axial magnetic bearing, which is generated as a result of mutual attraction between drive magnets and the ferromagnetic part of the stator, usually made of metal sheets or sintered materials. The phenomenon of mutual attraction between drive magnets and ferromagnetic metal sheets of the stator occurs both in the post-axial and in the transverse directions. Thus, using calculations, relationships between the values of forces acting in the post-axial and in the transverse direction are selected, enabling simultaneous operation of the post-axial and the transverse system without generating forces disturbing the operation of these systems.

Correct operation of the transverse hydrodynamic bearing system is possible only if at least equilibrium is achieved between hydrodynamic forces of the bearing and forces destabilising rotor position, and in particular, forces related to dynamic phenomena and forces occurring as a result of interactions between drive magnets and ferromagnetic elements of the motor and interactions between drive magnets and motor coils.

An advantage of the drive-bearing unit according to the invention is using of drive system elements to act as a post-axial bearing and a transverse bearing simultaneously, thus significantly simplifying the design of the flow duct which may be made as a monolithic element and allowing a smaller number of design elements to be used with increased pump reliability, a key element of a device mechanically supporting heart functions. The invention allows omitting a hydrodynamic post-axial bearing in its design. Thanks to the forced, continuous flow washing the rotor inside the gap between the fixed duct wall and the rotor, the solution according to the invention decreases the risk of embolism. In addition, by forming a stable hydrodynamic bearing, the invention allows the magnetic field intensity, used to provide contactless rotor bearing to be decreased and/or the power requirements of the pump to be reduced. Thus defined geometry enables the distance between stator coils and the rotor body to be decreased, whilst increasing the active surface area of rotor blades and maintaining the rotation speed at a level of ca. 6,000 RPM.

The invention is explained in more detail in embodiments presented in the drawing, in which Fig. 1 presents a hybrid drive-bearing unit with a stator with coils on teeth in two projections - transverse cross-section and in a longitudinal half-section, Fig. 2 presents geometry of the stator with teeth in three embodiments, Fig. 3 presents a hybrid drive-bearing unit with a stator without teeth in two projections - transverse cross-section and in a longitudinal half-section, and Fig. 4 schematically presents the location of the hybrid drive-bearing unit inside the pump supporting heart function.

Example I

In the example, the unit according to the invention is used in a single stage pump with a rotor provided with magnetic bearing using a system of passive or active bearings, or of passive and active bearings. The pump rotor is driven by a rotary electromagnetic field.

The design of the drive-bearing unit of pump rotor 1 includes a fixed flow duct 4 with a constant internal and external diameter, inside which the pump rotor 1 is placed. The rotor 1 rotates around the pump axis or rotates eccentrically around the flow duct 4 axis, inside which drive magnets 2 or a single drive magnet formed as a ring are provided. The stator with coils 3 wound onto its teeth is installed snug fitted on the flow duct bushing 4, above drive magnets 2. The rotor is made of a rotor body, on the inside of which blades 5 with constant or variable pitch are installed, integrated with the rotor body 1 and placed on a pin 6. In an example embodiment of the device, rotor rotation is generated using a brushless motor including a stator, coils provided in an at least three phase system and drive magnets.

The design parameters of the motor are directly related to design parameters of the transverse hydrodynamic bearing, formed as a result of rotation of the rotor submerged in the working medium, enabling formation of a stable liquid film in the gap between the wall of the flow duct 4 and the rotor body 1, resulting in generation of forces counteracting adhesion of the rotor to the flow duct wall. In an example embodiment of the device, forces resulting from magnetic phenomena present in the drive system are oriented transversely, and thus destabilise the rotor and are at least balanced by stabilising forces generated by the hydrodynamic bearing. Magnetic forces present in the drive system, oriented in the post-axial direction, are adequate to maintain the rotor in its working area and protect the rotor against leaving this area. Selection of design parameters of the magnetic circuit and of the flow system geometry is of key importance in terms of ensuring generation of the required drive moment while retaining a rotation speed determining generation of forces stabilising the rotor in the post-axial and in the transverse directions. Hydrodynamic forces generated in the transverse hydrodynamic bearing system do not cause shearing of the blood ingredients.

Depending on the design requirements of the pump, such as rotor rotation speed, drive moment, working temperature, power demand, fixture moment fluctuations, parameters of the drive-bearing system, such as the number of coils per single winding phase, number of turns of a single coil, number of phases, external stator diameter, stator length, drive magnet length and thickness are calculated.

Relationships between individual elements of the units are determined using the following dimensions:

- D0 determining internal diameter of the rotor body 1,
- D1 determining the internal diameter of drive magnets 2 or of the single drive magnet 2,
- D2 determining the external diameter of drive magnets 2 or of the single drive magnet 2,
- D3 determining external diameter of the rotor body 1,
- D4 determining internal diameter of the flow duct 4,
- D5 determining external diameter of the flow duct 4,
- D6 determining diameter of stator 3 tooth connection with the stator 3 ring,
- D7 determining external diameter of the stator 3 ring,
- D8 determining internal diameter of the stator 3 ring (for a toothless stator, although this diameter is also present in the toothed variant)

and have the values, determined as individual values in a range, according to the following relationships:

$$\frac{D7}{D5 + D2 - D1} = 1{,}4 \div 1{,}7$$

$$\frac{D3}{D0} = 1{,}1 \div 1{,}3$$

$$\frac{D7}{D4} = 1{,}4 \div 2{,}0 \quad .$$

The drive magnets 2 used are neodymium-based magnets available with the following designations: N30, N33, N35, N38, N40, N42, N45, N48, N50, N52, N55, N33M, N35M, N38M, N40M, N42M, N45M, N48M, N50M, N52M, N30H, N33H, N35H, N38H, N40H, N42H, N45H, N48H, N50H, N30SH, N33SH, N35SH, N38SH, N40SH, N42SH, N45SH, N48SH, N28UH, N30UH, N33UH, N35UH, N38UH, N40UH, N42UH, N45UH, N28EH, N30EH, N33EH, N35EH, N38EH, N40EH, N42EH, N28AH, N30AH, N33AH, N35AH, N38AH, N35X, N35MX, N45MX, N38HX, N40HX, N45HX, N33SHX, N35SHX, N38SHX, N42SHX, N30SHZ, N33SHZ, N35SHZ, N38SHZ, N45SHZ, N30UHZ, N33UHZ, N38UHZ, N40UHZ, N30EHZ, N38EHZ, N30AHZ, or magnets made of other alloys with energy density corresponding to energy density of the aforementioned magnets, e.g. samarium-cobalt magnets.

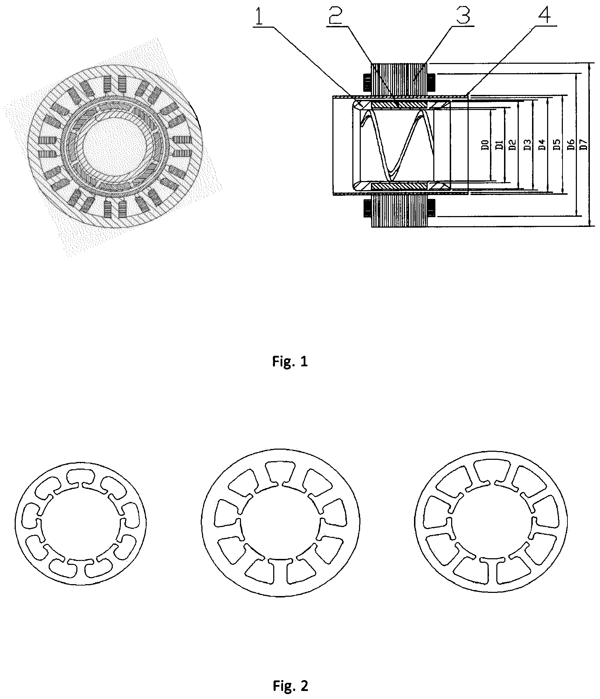

In the example according to Fig. 1 individual dimensions of unit elements are as follows:

- D0 = 14,32 mm,

- D1 = 15,06 mm,

- D2 = 17,46 mm,

- D3 = 18,09 mm,

- D4 = 19,05 mm,

- D5 = 19,85 mm,

- D6 = 28,79 mm,

- D7 = 32,81 mm.

The table presents rated flow rates of the pump supporting heart function, with a drive-bearing unit according to the invention.

| Rotor body external diameter [mm] | Range of the rated rotation speed of the rotor [L/min] | Rated flow rate [L/min] (for a pressure difference between the pump inlet of 100 mmHg) |
|---|---|---|
| 22,1 | 4 000-6 000 | 5 |
| 14,8 | 6 000-8 000 | 3 |

Example II

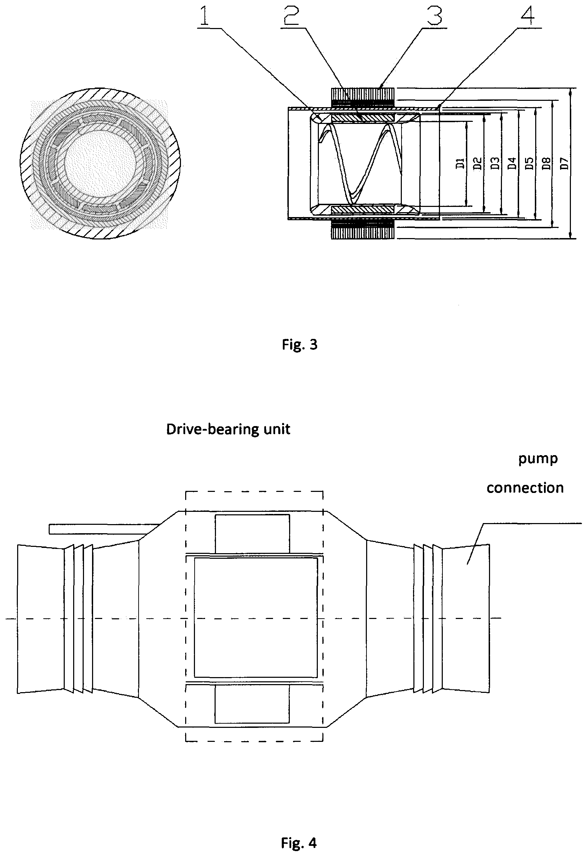

As fig. 3 shows, the unit is constructed as in Example I, wherein coils are placed between the toothless stator and the external part of the flow duct 4.

**Claims**

1. An axial-flow pump with a hybrid drive-bearing unit of its rotor, in particular a pump supporting heart functions, the pump comprising a ring-shaped stator provided with teeth with coils wound thereon or with coils placed on the inside, placed on a cylindrical bushing of the flow duct, inside which a rotor with blades is installed, wherein the blades are installed on the inside of the cylindrical rotor body, with at least one permanent drive magnet installed on the body, preferably a neodymium-based magnet, wherein the bearing system is formed in the post-axial direction as a result of magnetic interaction forces between the stator and the drive magnet or magnets, wherein the internal diameter (D4) of the flow duct bushing (4) is constant along the length of the rotor body (1), increased by the range of working, post-axial shift of the rotor, whilst the external diameter (D3) of the rotor body (1) is constant along its entire length and the thickness (D2-D1) of the drive magnet (2) is also constant, **characterised in that** the internal diameter (D4) of the flow duct bushing (4) is determined as one of the values in the range between 0,50 and 0,7 of the external diameter (D7) of the stator (3) ring (5), **in that** the external diameter (D3) of the rotor body (1) is determined as one of the values in the range between 1,1 and 1,3 of the internal diameter (D0) of the rotor body (1), **in that** the external diameter (D7) of the stator ring (3) is between 1,4 and 1,7 of the sum of external diameter (D5) of the flow duct bushing (4) and of the drive magnet (2) thickness, and **in that** the thickness (D2-D1) of the drive magnet (2) is determined as one of the values in the range determined using the following relationship:

$$0{,}55 \leq \frac{D2 + D1}{D6 + D5 + D2 - D1} \leq 0{,}7$$

where

D1 - internal diameter of drive magnets or of the single drive magnet,
D2 - external diameter of drive magnets or of the single drive magnet,
D5 - external diameter of the flow duct bushing (4),
D6 - internal diameter of the stator (3) ring (5),

2. A pump according to claim 1 **characterised in that** the rotor body (1) is provided with circumferentially bevelled edges.

**Patentansprüche**

1. Axialpumpe mit einer hybriden Rotorantriebslageranordnung, nämlich eine Pumpe, die die Arbeit des Herzens unterstützt, Pumpe bestehend aus einem ringförmigen Stator mit Wicklung auf der Verzahnung oder auf der Innenseite, der sich auf einer zylindrischen Hülse des Strömungskanals befindet, in der ein Rotor mit Schaufeln installiert ist, wobei die Schaufeln auf der Innenseite des zylindrischen Rotorkörpers installiert sind und sich auf dem Körper mindestens ein Permanentmagnet des Motors befindet, vorzugsweise ein Neodym-Magnet und das Lagersystem, infolge der Wechselwirkung magnetischer Kräfte zwischen dem Stator und dem Motormagnet oder den Motormagneten, in axialer Richtung geformt wird, wobei der Innendurchmesser (D4) der Strömungskanalhülse (4) entlang des Rotorkörpers (1), erhöht um den Bereich der Axialverschiebung des Rotors im Betrieb, konstant ist, dagegen der Außendurchmesser (D3) des Rotorkörpers (1) in Längsrichtung konstant ist und die Dicke (D2-D1) des Motormagnets (2) ebenfalls konstant ist, wobei charakteristisch ist, dass der Innendurchmesser (D4) der Strömungskanalhülse (4) auf einen der Werte im Bereich von 0,50 bis 0,7 des Außendurchmessers (D7) des Rings (5) des Stators (3) eingestellt ist, weil der Außendurchmesser (D3) des Rotorkörpers (1) auf einen der Werte im Bereich von 1,1 bis 1,3 des Innendurchmessers (D0) des Rotorkörpers (1) festgelegt ist, da der Innendurchmesser (D7) des Rings des Stators (3) von 1,4 bis 1,7 der Summe aus dem Außendurchmesser (D5) der Strömungskanalhülse (4) und der Dicke des Motormagnets (2) beträgt, und da auch die Dicke (D2-D1) des Motormagnets (2) als einer der Werte im ermittelten Bereich anhand folgender Abhängigkeit festgelegt wird:

$$0,55 \le \frac{D2+D1}{D6+D5+D2-D1} \le 0,7$$

wobei

D1 - Innendurchmesser der Motormagnete oder eines einzelnen Motormagnets,
D2 - Außendurchmesser der Motormagnete oder eines einzelnen Motormagnets,
D5 - Außendurchmesser der Strömungskanalhülse (4),
D6 - Innendurchmesser des Rings (5) des Stators (3).

2. Die Pumpe gemäß Anspruch 1 ist **dadurch gekennzeichnet, dass** der Rotorkörper (1) mit kreisförmig abgeschrägten Kanten hergestellt ist.


**Revendications**

1. Pompe axiale avec un ensemble de palier d'entraînement de rotor hybride, à savoir une pompe d'assistance cardiaque, une pompe constituée d'un stator en forme d'anneau avec l'enroulement sur les dents ou à l'intérieur, situé sur le manchon cylindrique du canal d'écoulement, à l'intérieur duquel est installée un rotor avec les pales, où les pales sont installées à l'intérieur du corps de rotor cylindrique, et sur ce corps il y a au moins un aimant permanent du moteur, de préférence de néodyme, et le système de palier à la suite de l'interaction des forces magnétiques entre le stator et l'aimant ou les aimants du moteur est formé dans la direction axiale où le diamètre intérieur (D4) du manchon de canal d'écoulement (4) le long du corps de rotor (1), augmenté de la plage de déplacement axial du rotor en fonctionnement est constante, tandis que le diamètre extérieur (D3) du corps de rotor (1) est constant dans la direction longitudinale et l'épaisseur (D2-D1) de l'aimant du moteur (2) est également constante, et il est caractéristique que le diamètre intérieur (D4) du manchon de canal d'écoulement (4) est établie comme l'une des valeurs comprises entre 0,50 et 0,7 du diamètre extérieur (D7) de l'anneau (5) de stator (3), étant donné que le diamètre extérieur (D3) du corps de rotor (1) est fixé comme l'une des valeurs comprises entre 1,1 et 1,3 du diamètre extérieur (D0) du corps de rotor (1), car le diamètre extérieur (D7) de l'anneau de stator (3) est de 1,4 à 1,7 de la somme du diamètre extérieur (D5) du manchon de canal d'écoulement (4) et de l'épaisseur de l'aimant du moteur (2) et aussi parce que l'épaisseur (D2-D1) de l'aimant du moteur (2) est définie comme l'une des valeurs de la plage déterminée en fonction suivante:

$$0,55 \le \frac{D2+D1}{D6+D5+D2-D1} \le 0,7$$

où

D1 - le diamètre intérieur des aimants du moteur ou d'un seul aimant du moteur,
D2 - le diamètre extérieur des aimants du moteur ou d'un seul aimant du moteur,
D5 - le diamètre extérieur du manchon du canal d'écoulement (4),
D6 - le diamètre intérieur de l'anneau (5) de stator (3).

2. La pompe selon la revendication 1 est **caractérisée en ce que** le corps de rotor (1) est réalisé avec des bords chanfreinés circulairement.

**Fig. 1**

**Fig. 2**

Fig. 3

Drive-bearing unit

pump
connection

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6527521 B
- WO 2007003351 A
- US 8790236 B
- US 5211546 A
- WO 2017004175 A1

### Non-patent literature cited in the description

- **WILCZYŃSKI L. ; DITRICH M. ; HOFFMANN P. ; DAWIDOWSKA K. ; BOROWIECKI B. ; GÓRSKI W. ; JAKUBOWSKI P. ; SIONDALSKI P. ; SMOLENSKI R.** *Opracowanie konstrukcji osiowej pompy wspomagania serca,* 02 April 2015, www.researchgate.net